# FASCICULE DE BREVET EUROPEEN

(11) **EP 1 529 518 B1**
(45) Date de publication et mention de la délivrance du brevet: **13.08.2008**
(21) Numéro de dépôt: 04292617.0
(22) Date de dépôt: 04.11.2004
(51) Int. Cl.: A61Q 5/06, A61Q 5/10, A61K 8/40, A61K 8/37

(54) **Composition comprenant un derivé de type heptafulvène ou tropolone et procédé de teinture des cheveux**
Zusammensetzung, die ein Derivat des Typs Heptafulven oder Tropolon enthält und Verfahren zur Färbung der Haare
Composition containing a derivative of the type heptafulvene or tropolone and process for hair dyeing

(30) Priorité: 05.11.2003 FR 0350788
(43) Date de publication de la demande: 11.05.2005
(73) Titulaire: L'ORÉAL, 75008 Paris (FR)
(72) Inventeur: Gourlaouen, Luc, 92600 Asnières (FR); Samain, Henri, 91570 Bievres (FR)
(74) Mandataire: Wattremez, Catherine

(56) Documents cités:
- DE-A- 3 508 896
- DE-A- 19 717 225
- PATENT ABSTRACTS OF JAPAN vol. 1998, no. 14, 31 décembre 1998 (1998-12-31) & JP 10 245498 A (MITSUBISHI CHEM CORP), 14 septembre 1998 (1998-09-14)
- PATENT ABSTRACTS OF JAPAN & JP 02 138114 A (KASHIWA KAGAKU KOGYO), 28 mai 1990 (1990-05-28)
- CHEMICAL ABSTRACTS, Columbus, Ohio, US; abstract no.: 1999:37503, JIN MIZUGUCHI ET AL: "A novel NLO-active, non-benzenoid compound based on 8,8-dicyano-3-(4'-dimethylamino)-phenylhep tafulvene; crystals and electronic structures"

## Description

La présente invention a pour objet une composition destinée à la coloration des fibres kératiniques, comprenant dans un milieu approprié, au moins un composé dérivé de type heptafulvène, ou leurs dérivés ainsi que leurs mélanges, de même qu'un procédé de coloration la mettant en oeuvre.

L'invention a donc trait au domaine de la coloration des fibres kératiniques, notamment humaines, et en particulier des cheveux. Plus particulièrement, elle concerne les colorations obtenues au moyen de compositions tinctoriales comprenant au moins un colorant direct.
Les colorants directs sont des substances colorées et colorantes qui ont une certaine affinité avec les fibres kératiniques humaines. Parmi les colorants directs utilisables, on peut citer la classe des colorants benzéniques nitrés, les colorants directs hétérocycliques, notamment, qui sont utilisés pour obtenir non seulement des couleurs naturelles, mais aussi des couleurs chromatiques.
Même si ces colorants ont été utilisés de manière importante dans le domaine de la coloration capillaire, ils peuvent malgré tout poser des problèmes de montée de couleur dans la fibre, et notamment au niveau de la puissance et de la sélectivité obtenues. Par ailleurs, la ténacité de ces colorants peut encore être améliorée.

Il est connu d'après les demandes de brevet DE 35 08896, DE 197 17 225 de colorer des fibres kératiniques en mettant en oeuvre des compositions tinctoriales comprenant des dérivés particuliers de tropolone, ainsi que d'employer des composés naturels dont l'hinokitiol (JP 02 138114).
Par ailleurs, d'après JP 10245498 et la publication Chem Abst 1999:37503 des composés de type heptafulvène portant des groupements cyano sont connus mais employés dans un domaine totalement différent de celui de la cosmétique, et en particulier de la coloration capillaire, l'optique non linéaire.
Ainsi, la présente invention a pour objet de proposer des compositions comprenant au moins un colorant direct permettant d'obtenir de meilleurs résultats de coloration que les colorants directs benzéniques et hétérocycliques.
Ces buts et d'autres sont atteints par la présente invention qui a donc pour premier objet une composition comprenant, dans un milieu approprié pour la teinture des fibres kératiniques, notamment humaines, au moins un composé de formule (I) suivante : 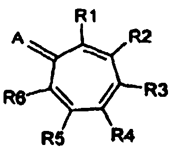 Formule dans laquelle :
* R₁ et R₆ identiques ou non, représentent un atome d'hydrogène ; un radical alkyle, un radical aryle, un radical alkylaryle, un radical arylalkyle, pour lesquels la partie alkyle est en C₁-C₆, linéaire ou ramifiée, substituée ou non, la partie aryle est en C₆-C₁₂, substituée ou non ; un atome d'halogène ; un radical alcoxy en C₁-C₆ ; un radical amino ; un radical amino substitué par un ou plusieurs radicaux, identiques ou non, choisis parmi les radicaux alkyles en C₁-C₆, linéaires ou ramifiés, substitués ou non ; un radical hydroxyle ; un radical -COOX, avec X représentant un atome d'hydrogène, un métal alcalin ou alcalino-terreux, un radical alkyle en C₁-C₆, linéaire ou ramifié, substitué ou non ; un radical alkylcarbonyle dans lequel la partie alkyle est en C₁-C₆, linéaire ou ramifié, substitué ou non ; un radical -SO₃X' dans lequel X' désigne un atome d'hydrogène, un métal alcalin ou alcalino-terreux ;
* R₂, R₃, R₄ et R₅, identiques ou non, représentent un atome d'hydrogène, un radical alkyle en C₁-C₆, linéaire ou ramifié, substitué ou non ; un radical hydroxyle ; un radical alcoxy en C₁-C₆ ; un radical thiol, un radical alkylthio en C₁-C₆ ; un radical amino ; un radical amino substitué par un ou plusieurs radicaux alkyles, identiques ou non, en C₁-C₆, linéaires ou ramifiés, substitués ou non ; un radical aryle en C₆-C₁₂, substitué ou non, un radical arylalkyle dont la partie alkyle est en C₁-C₆, linéaire ou ramifiée, substituée ou non, et la partie aryle est en C₆-C₁₂ ; un radical cyclique ou polycyclique, substitué ou non, comprenant au moins un hétéroatome ;
* A désigne un groupement C(R₇)(R₈) dans lequel R₇ et R₈, désignent un radical cyano;
Elle concerne de plus une composition prête à l'emploi comprenant la composition selon l'invention et au moins un agent oxydant.
Elle a de même pour objet un procédé de coloration des fibres kératiniques humaines utilisant les compositions qui viennent d'être décrites, ainsi qu'un dispositif pour la mise en oeuvre dudit procédé.
Elle a enfin pour objet l'utilisation de composés de formule (I) comme colorant direct pour la coloration de fibres kératiniques, notamment humaines afin d'obtenir des nuances naturelles et chromatiques.
Il a en effet été trouvé de manière totalement inattendue que la présence de composés de formule (I) permettait d'obtenir des nuances naturelles et chromatiques, qui soient notamment puissantes et peu sélectives.

Mais d'autres caractéristiques et avantages de la présente invention apparaîtront plus clairement à la lecture de la description et des exemples qui vont suivre.

Dans ce qui va suivre, les teneurs des divers composés présents dans la composition sont exprimées en poids par rapport au poids de la composition, à moins qu'il ne soit précisé que ces teneurs le sont par rapport au poids de la composition prête à l'emploi. Il est rappelé que la composition prête à l'emploi comprend la composition selon l'invention et au moins un agent oxydant.
Par ailleurs, sauf indication différente, les bornes délimitant un domaine de valeurs sont comprises dans ce domaine.
Enfin, les fibres kératiniques traitées conformément à l'invention, sont plus particulièrement des fibres kératiniques humaines et de préférence des cheveux.

La composition selon l'invention comprend donc au moins un composé de formule (I) précédemment indiquée.
Plus particulièrement, dans cette formule (I), R₁ et R₆ identiques ou non, représentent un atome d'hydrogène ; un radical alkyle, un radical aryle, un radical alkylaryle, un radical arylalkyle, pour lesquels la partie alkyle est en C₁-C₆, linéaire ou ramifiée, substituée ou non, la partie aryle est en C₆-C₁₂, de préférence en C₆, substituée ou non ; un atome d'halogène ; un radical alcoxy en C₁-C₆ ; un radical amino ; un radical amino substitué par un ou plusieurs radicaux, identiques ou non, choisis parmi les radicaux alkyles en C₁-C₆, linéaires ou ramifiés, substitués ou non ; un radical hydroxyle ; un radical -COOX, avec X représentant un atome d'hydrogène, un métal alcalin ou alcalino-terreux, un radical alkyle en C₁-C₆, linéaire ou ramifié, substitué ou non ; un radical alkylcarbonyle dans lequel la partie alkyle est en C₁-C₆, linéaire ou ramifié, substitué ou non ; un radical -SO₃X' dans lequel X' désigne un atome d'hydrogène, un métal alcalin ou alcalino-terreux, comme par exemple le sodium, le potassium, le magnésium, le calcium.
Comme indiqué auparavant, dans la formule (I), R₂, R₃, R₄ et R₅, identiques ou non, représentent un atome d'hydrogène, un radical alkyle en C₁-C₆, linéaire ou ramifié, substitué ou non ; un radical hydroxyle ; un radical alcoxy en C₁-C₆ ; un radical thiol, un radical alkylthio en C₁-C₆ ; un radical amino ; un radical amino substitué par un ou plusieurs radicaux alkyle, identiques ou non, en C₁-C₆, linéaires ou ramifiés, substitués ou non ; un radical aryle en C₆-C₁₂, de préférence en C₆, substitué ou non, un radical arylalkyle dont la partie alkyle est en C₁-C₆, linéaire ou ramifiée, substituée ou non, et la partie aryle est en C₆-C₁₂, de préférence en C₆ ; radical cyclique ou polycyclique, substitué ou non, comprenant au moins un hétéroatome.

Sauf indication contraire, on dit qu'un radical est substitué, lorsqu'il porte un ou plusieurs groupements choisis parmi :
□ un groupement hydroxyle ;
□ un atome d'halogène, et de préférence le chlore, le fluor ;
□ un groupement alcoxy en C₁-C₆ ;
□ un groupement monohydroxy alcoxy dont la partie alkyle en C₁-C₆, linéaire ou ramifiée, substituée ou non ;
□ un groupement polyhydroxy alcoxy, dans lequel la partie alkyle est en C₂-C₆, linéaire ou ramifiée, substituée ou non ;
□ un groupement amino substitué ou non par un ou plusieurs radicaux alkyles en C₁-C₆, identiques ou non, linéaires ou ramifiés, substitué ou non ;
□ un groupement thiol ;
□ un groupement alkylthio dans lequel la partie alkyle est en C₁-C₆, linéaire ou ramifiée, substituée ou non ;
□ un groupement carboxylique sous forme acide ou salifiée (par exemple avec un métal alcalin ou un ammonium substitué ou non) ;
□ un groupement alkylcarbonyle dont la partie alkyle est en C₁-C₆, linéaire ou ramifiée, substituée ou non ;
□ un groupement alcoxycarbonyle dont la partie alkyle est en C₁-C₆, linéaire ou ramifiée, substituée ou non ;
□ un groupement alkylamide dont la partie alkyle est en C₁-C₆, linéaire ou ramifiée, substitué ou non ;
□ un groupement alkylcarbamyle dont la partie alkyle est en C₁-C₆, linéaire ou ramifiée, substituée ou non ;
□ un groupement cyano ;
□ un groupement nitro ;
□ un groupement sulfonyle ;
□ un groupement alkylsulfonyle dont la partie alkyle est en C₁-C₆, linéaire ou ramifiée, substituée ou non ;
□ un groupement alkylsulfonylamido dont la partie alkyle est en C₁-C₆, linéaire ou ramifiée, substituée ou non ;
□ un radical alkyle si le radical substitué est lui-même différent d'un radical alkyle.

Il est rappelé que les parties alkyles des groupements mentionnés ci-dessus peuvent, elles aussi, être substituées par un ou plusieurs des groupements listés. Cependant, conformément à un mode de réalisation particulier de l'invention, les parties alkyles desdits groupements ne sont pas substituées.

En ce qui concerne les radicaux cycliques ou polycycliques comprenant au moins un hétéroatome, ces derniers comprennent de 5 à 8 chaînons, de préférence de 5 à 6 chaînons.
Par ailleurs, le ou les hétéroatomes présents, identiques ou non, sont choisis parmi l'oxygène, l'azote ou le soufre.
De plus, ces radicaux cycliques ou polycycliques peuvent être saturés ou non, ou aromatiques.
A titre d'exemples, on peut citer les cycles pyrrolidinique, pipéridinique, pipérazinique, pyrazolique, imidazolique, triazolique, oxazolique, thiazolique, pyridinique, pyrimidinique, pyrazinique, pyridazinique.

Selon une variante avantageuse de l'invention, R₁, R₆, identiques ou non, sont choisis parmi l'hydrogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical alkylcarbonyle en C₁-C₆, un radical amino, un radical amino mono- ou di-substitué par un radical alkyle en C₁-C₆ et/ou par un radical hydroxyalkyle en C₁-C₆ ; un radical benzyle ou phényle éventuellement substitué au sens indiqué auparavant ; un groupement nitro ; un groupement cyano.

Conformément à une autre variante avantageuse de l'invention, les radicaux R₂ à R₅, identiques ou non, sont choisis parmi l'hydrogène ; un radical hydroxyle ; un radical thiol ; un atome d'halogène et de préférence le chlore ou le fluor ; un radical alkyle en C₁-C₆ ; un radical monohydroxyalkyle en C₁-C₆ ; un radical polyhydroxyalkyle en C₂-C₆ ; un radical alcoxy en C₁-C₆ ; un radical alkylcarbonyle en C₁-C₆ ; un radical amino ; un radical amino mono- ou di-substitué par un radical alkyle en C₁-C₆ et/ou par un radical hydroxyalkyle en C₁-C₆ ; un radical benzyle ou phényle éventuellement substitué au sens indiqué auparavant ; un groupement nitro ; un groupement cyano ; un groupement hétérocyclique.

Un des radicaux R₃ ou R₄ représente plus particulièrement un radical aryle en C₆-C₁₂, substitué ou non par un radical hydroxyle ; un atome d'halogène ; un radical alcoxy en C₁-C₄ ; un radical amino ; un radical amino substitué par un ou plusieurs radicaux alkyles, identiques ou non, en C₁-C₄ ; un radical thiol. De manière avantageuse, l'autre radical représente un atome d'hydrogène.

De préférence, au moins l'un des radicaux R₁, R₂, R₅ et R₆, dans le cadre de ce mode de réalisation, représentent un atome d'hydrogène. De manière avantageuse les radicaux R₁, R₂, R₅ et R₆, représentent un atome d'hydrogène.
Parmi les composés préférés entrant dans le cadre de ce deuxième mode de réalisation, on peut citer entre autres, le 8,8-dicyano-3-(4'-méthoxy)-phénylheptafulvène ; le 8,8-dicyano-3-(4'-N,N-diméthylamino)-phénylheptafulvène ; le 8,8-dicyano-3-phényl heptafulvène, ou leurs mélanges.
De préférence, le composé de formule (I) se trouve sous la forme d'un colorant. Ainsi, le composé est présent en quantité telle qu'il se trouve sous une forme soluble dans le milieu de la composition. Plus particulièrement, le composé est soluble à au moins 50 % en poids dans le milieu, de préférence, le composé est soluble à au moins 90 % en poids dans le milieu.
La teneur en composé (I) présent dans la composition selon l'invention est plus particulièrement comprise entre 0,1 et 20 % en poids par rapport au poids de la composition, de préférence entre 0,5 et 5 % en poids par rapport au poids de la composition.
La composition peut renfermer d'autres ingrédients classiques dans le domaine des compositions tinctoriales destinées à la coloration des fibres kératiniques humaines, notamment des cheveux.
Ainsi, elle peut comprendre au moins un colorant direct, fluorescent ou non, différent de celui qui vient d'être décrit.
Notons que les colorants directs peuvent être choisis parmi les espèces non ioniques ou ioniques (cationiques, anioniques, amphotères ou zwitterioniques), et de préférence non ioniques ou cationiques.
A titre d'exemples non limitatifs, on peut citer les colorants directs benzéniques nitrés, les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (notamment les azacarbocyanines, diazacarbocyanines, diazahémicyanines, hémicyanines, tétraazacarbocyanines), diazines, dicétopyrrolopyrroles, dioxazines, diphénylamines, diphénylméthanes, dithiazines, flavanthrones, flavones, fluorindines, formazans, hydrazones, hydroxycétones, indamines, indanthrones, indigoides, indophénols, indoanilines, isoindolines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes.
Conviennent aussi les colorants directs fluorescents choisis parmi les composés fluorescents cationiques de la famille des composés azoïques, azométhiniques, méthiniques, naphtalimides, coumarines cationiques ou non, xanthénodiquinolizines, les azaxanthènes, naphtolactames, azlactones, (di)oxazines, thiazines, seuls ou en mélanges.
Si la composition comprend ce type de colorant direct, alors la teneur en colorant(s) direct(s), fluorescent(s) ou non, représente avantageusement de 0,0005 à 12 % en poids par rapport au poids de la composition, et encore plus préférentiellement de 0,005 à 6 % en poids par rapport au poids de la composition.
La composition selon l'invention peut aussi comprendre au moins une base d'oxydation éventuellement associée à un ou plusieurs coupleurs.
La base d'oxydation peut être choisie parmi les composés classiques dans le domaine de la coloration.
A titre d'exemples, on peut citer entre autres les o-phénylènediamines, les p-phénylènediamines, les bases doubles, les o-aminophénols, les p-aminophénols, les bases hétérocycliques, leurs sels d'addition avec un acide, ainsi que leurs mélanges.
Si la composition comprend une ou plusieurs bases d'oxydation, la teneur en ce type de composé représente habituellement de 0,0005 à 12% en poids par rapport au poids de la composition, et de préférence de 0,005 à 8% en poids par rapport au poids de la composition.
Parmi les coupleurs utilisables en association avec une ou plusieurs bases d'oxydation, on peut mentionner par exemple les m-aminophénols, les m-phénylènediamines, les m-diphénols, les naphtols, les coupleurs hétérocycliques, leurs sels d'addition avec un acide, ainsi que leurs mélanges.
Au cas où ils sont présents dans la composition, leur teneur représente en général de 0,0001 à 10% en poids par rapport au poids de la composition, et de préférence de 0,005 à 5% en poids par rapport au poids de la composition.
D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut également comprendre divers adjuvants utilisés classiquement, tels que des agents tensioactifs anioniques, cationiques, non ioniques, amphotères, zwittérioniques ou leurs mélanges ; des polymères anioniques, cationiques, non ioniques, amphotères, zwittérioniques ; des agents épaississants minéraux ; des agents antioxydants ; des agents de pénétration ; des parfums ; des tampons ; des agents dispersants ; des agents de conditionnement tels que par exemple des cations, des silicones volatiles ou non volatiles, modifiées ou non modifiées ; des polymères amphiphiles cationiques ; des agents filmogènes ; des céramides ; des vitamines ou provitamines ; des agents conservateurs ; des agents stabilisants ; des agents opacifiants ou matifiants comme le dioxyde de titane ; des charges minérales, comme les argiles ; les silices notamment pyrogénées à caractère hydrophile ou hydrophobe ; des polymères liants tels que la vinylpyrrolidone ; des filtres solaires ; etc.

Les adjuvants cités ci-dessus sont en général présents en quantité comprise pour chacun d'eux entre 0,01 et 20% en poids par rapport au poids de la composition.

Le milieu de la composition est un milieu cosmétiquement acceptable.
Il est constitué, de manière avantageuse, par de l'eau ou par un mélange d'eau et d'un ou plusieurs solvants organiques.
Parmi les solvants organiques usuels, on peut citer notamment les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.
Les solvants organiques décrits ci-dessus, s'ils sont présents, représentent habituellement de 1 à 40 % en poids, plus préférentiellement de 5 à 30 % en poids, par rapport au poids total de la composition.

Le pH de la composition selon l'invention est généralement compris entre 3 et 12 environ, et de préférence entre 5 et 11 environ.
Il peut être ajusté à la valeur désirée au moyen d'agents acidifiants ou alcalinisants.
Parmi les agents acidifiants, on peut citer, à titre d'exemples, les acides minéraux ou organiques comme l'acide chlorhydrique, l'acide orthophosphorique, l'acide sulfurique, l'acide acétique.
Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 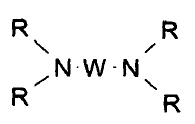 dans laquelle W est un reste propylène éventuellement substitué par un groupement hydroxyle ou un radical alkyle en C₁-C₆ ; les radicaux R, identiques ou différents, représentent un atome d'hydrogène, un radical alkyle en C₁-C₆ ou hydroxyalkyle en C₁-C₆.

Notons que la composition peut se trouver sous la forme d'une lotion épaissie ou non, d'une crème, d'un gel, d'une mousse, d'un spray, ou de toute autre forme appropriée pour l'application ultérieure de cette composition.

Un autre objet de la présente invention est constitué par une composition prête à l'emploi comprenant la composition selon l'invention qui vient d'être détaillée et au moins un agent oxydant.
Habituellement, l'agent oxydant est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges. L'agent oxydant peut aussi être choisi parmi les enzymes telles que les peroxydases et les oxydo-réductases à deux ou à quatre électrons.
La teneur en agent oxydant représente 0,001 et 20% en poids par rapport au poids de la composition prête à l'emploi, de préférence 0,1 à 12 % en poids par rapport au poids de la composition prête à l'emploi.
Le pH de la composition prête à l'emploi est généralement compris entre les 4 et 12, de préférence entre 6 et 11.
Il peut être ajusté à la valeur désirée au moyen d'agents acidifiants ou alcalinisants tels que ceux décrits auparavant.
La composition prête à l'emploi résulte plus particulièrement du mélange de la composition selon l'invention avec une composition comprenant au moins un agent oxydant (appelé composition oxydante). Dans ce cas, le mélange est réalisé avant l'application de la composition prête à l'emploi sur les fibres à traiter.
Le milieu approprié pour la teinture de la composition oxydante est avantageusement de l'eau ou un mélange d'eau et de solvant organique.
Enfin, la composition oxydante peut comprendre les additifs usuels dans le domaine, comme des agents tensioactifs, des agents épaississants, des agents anti-oxydants, des parfums, des agents dispersants, des agents de conditionnement, des agents séquestrants, des agents conservateurs, etc.
Précisons que les listes de solvants et d'additifs, de même que leurs teneurs, indiquées dans le cadre de la description de la composition selon l'invention restent valables et l'on peut s'y reporter.

Un autre objet de l'invention est constitué par un procédé de coloration des fibres kératiniques, notamment humaines et plus particulièrement des cheveux, dans lequel on applique la composition qui vient d'être détaillée sur lesdites fibres, sèches ou humides.
Plus particulièrement, l'invention est constitué par un procédé de coloration des fibres kératiniques humaines et notamment des cheveux, dans lequel on met en oeuvre les étapes suivantes :
a) on met en contact lesdites fibres, sèches ou humides, avec la composition selon l'invention pendant une durée suffisante pour le développement de la coloration,
b) on rince éventuellement les fibres,
c) éventuellement on lave les fibres et on les rince,
d) on sèche les fibres ou on les laisse sécher.

Conformément à une première possibilité, le procédé est mis en oeuvre sans rinçage de la composition. Ainsi, après l'application de la composition sur les fibres, celles-ci sont séchées ou laissées à sécher. Cette possibilité peut notamment être appropriée dans le cas où la composition ne comprend pas d'agent oxydant, ni de base d'oxydation ni de coupleur.
Conformément à une deuxième possibilité, le procédé est mis en oeuvre avec au moins une étape de rinçage.

Selon une première variante du procédé, la composition appliquée sur les fibres ne comprend pas d'agent oxydant.
Cette variante est particulièrement appropriée lorsque la composition ne comprend que le ou les composés de formule (I) et éventuellement a moins un colorant direct fluorescent ou non.
Selon une deuxième variante du procédé, la composition appliquée sur les fibres est une composition prête à l'emploi telle que décrite ci-dessus.
Cette variante du procédé est appropriée quelle que soit la composition selon l'invention mise en oeuvre, que celle-ci comprenne ou non, outre le composé de formule (I), un colorant direct fluorescent ou non, une ou plusieurs bases d'oxydation et éventuellement un ou plusieurs coupleurs.
Conformément à cette variante, une première possibilité consiste à appliquer la composition prête à l'emploi, obtenue par mélange extemporané avant l'application sur les fibres, de la composition selon l'invention et d'une composition oxydante.
Selon cette possibilité, il peut être avantageux de stocker sous forme séparée, d'une part, la composition selon l'invention, et d'autre part, une composition oxydante.
Une seconde possibilité consiste à appliquer successivement, dans un ordre ou dans l'autre, la composition selon l'invention et la composition oxydante, ou bien encore consiste à appliquer ces deux compositions simultanément.
La durée nécessaire au développement de la coloration est en général comprise entre 1 à 60 minutes, et plus préférentiellement entre 5 et 45 minutes.
Par ailleurs, de manière classique l'étape a) du procédé est effectuée à une température comprise entre 15 et 220°C, de préférence à une température comprise entre 15 et 40°C.
Une fois l'étape a) terminée, on peut éventuellement rincer les fibres (étape b), éventuellement les laver avec un shampooing puis les rincer, et enfin les sécher ou les laisser sécher, par exemple entre 20 et 120°C.

Des exemples concrets mais non limitatifs de l'invention vont maintenant être présentés.

### EXEMPLES

On prépare les formulations suivantes :

| **Matière première** | **Gramme %** |
|---|---|
| Colorant de formule (I) | 0,25 |
| Ethanol | Quantité nécessaire à la solubilisation du composé de formule (I) |
| Eau désionisée | Qsp 50 |

### Exemple 1 8,8-Dicyano-3-(4'-methoxy)-phénylheptafulvène de formule :

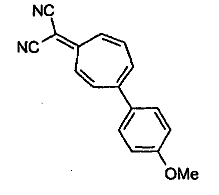

### Exemple 2 8,8-dicyano-3-(4'-N,N-dimethylamino)-phénylheptafulvène de formule :

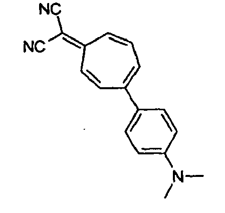

### Exemple 3 8,8-dicyano-3-phenyl heptafulvene de formule :

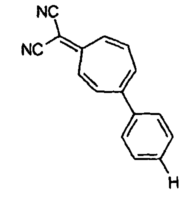

:

| **Exemples** | **Couleur du cheveu après coloration** |
|---|---|
| Exemple 1 | Orangé rouge |
| Exemple 2 | Noir violet |
| Exemple 3 | Orangé rouge |

## Revendications

1. Composition comprenant, dans un milieu approprié pour la teinture des fibres kératiniques, notamment humaines, de 0,01 à 20% en poids par rapport au poids de la composition , d' au moins un composé de formule (I) suivante : 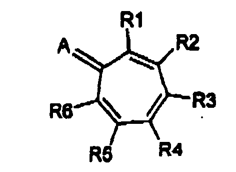 Formule dans laquelle:
* R₁ et R₆ identiques ou non, représentent un atome d'hydrogène : un radical alkyle, un radical aryle, un radical alkylaryle, un radical arylalkyle, pour lesquels la partie alkyle est en C₁-C₆, linéaire ou ramifiée, substituée ou non, la partie aryle est en C₆-C₁₂, substituée ou non ; un atome d'halogène ; un radical alcoxy en C₁-C₈ ; un radical amino : un radical amino substitué par un ou plusieurs radicaux, identiques ou non, choisis parmi les radicaux alkyles en C₁-C₈, linéaires ou ramifiés, substitués ou non ; un radical hydroxyle ; un radical -COOX, avec X représentant un atome d'hydrogène, un métal alcalin ou alcalino-terreux, un radical alkyle en C₁-C₆, linéaire ou ramifié, substitué ou non ; un radical alkylcarbonyle dans lequel la partie alkyle est en C₁-C₆, linéaire ou ramifié, substitué ou non ; un radical -SO₃X' dans lequel X' désigne un atome d'hydrogène, un métal alcalin ou alcalino-terreux ;
*R₂, R₃, R₄ et R₅, identiques ou non, représentent un atome d'hydrogène, un radical alkyle en C₁-C₆, linéaire ou ramifié, substitué ou non : un radical hydroxyle : un radical alcoxy en C₁-C₆ ; un radical thiol, un radical alkylthio en C₁-C₆ : un radical amino; un radical amino substitué par un ou plusieurs radicaux, identiques ou non, en C₁-C₆, linéaires ou ramifiés, substitués ou non ; un radical aryle en C₆-C₁₂, substitué ou non, un radical arylalkyle dont la partie alkyle est en C₁-C₆, linéaire ou ramifiée, substituée
ou non, et la partie aryle est en C₈-C₁₂ ; un radical cyclique ou polycyclique, substitué ou non, comprenant au moins un hétéroatome :
* A désigne un groupement C(R₇)(R₈) dans lequel R₇ et R₈, désignent un Radical cyano.

2. Composition selon la revendication précédente, **caractérisé en ce que** R₁ à R₆ sont tels que les radicaux substitués portent un ou plusieurs groupements choisis parmi :
□ un groupement hydroxyle;
□ un atome d'halogène, et de préférence le chlore, le fluor;
□ un groupement alcoxy en C₁-C₆ :
□ un groupement monohydroxy alcoxy dont la partie alkyle en C₁-C₆, linéaire ou ramifiée, substituée ou non ;
□ un groupement polyhydroxy alcoxy, dans lequel la partie alkyle est en C₂-C₈, linéaire ou ramifiée, substituée ou non ;
□ un groupement amino substitué ou non par un ou plusieurs radicaux alkyles en C₁-C₆, identiques ou non, linéaires ou ramifiés, substitué ou non ;
□ un groupement thiol;
□ un groupement alkylthio dans lequel la partie alkyle est en C₁-C₆, linéaire ou ramifiée, substituée ou non ;
□ un groupement carboxylique sous forme acide ou salifiée (par exemple avec un métal alcalin ou un ammonium substitué ou non) ;
□ un groupement alkylcarbonyle dont la partie alkyle est en C₁-C₆, linéaire ou ramiflée, substituée ou non ;
□ un groupement alcoxycarbonyle dont la partie alkyle est en C₁-C₆, linéaire ou ramifiée, substituée ou non ;
□ un groupement alkylamide dont la partie alkyle est en C₁-C₆, linéaire ou ramifiée, substitué ou non ;
□ un groupement alkylcarbamyle dont la partie alkyle est en C₁-C₆, linéaire ou ramifiée, substituée ou non :
□ un groupement cyano ;
□ un groupement nitro ;
□ un groupement sulfonyle ;
□ un groupement alkylsulfonyle dont la partie alkyle est en C₁-C₆, linéaire ou ramifiée, substituée ou non ;
□ un groupement alkylsulfonylamido dont la partie alkyle est en C₁-C₆, linéaire ou ramifiée, substituée ou non;
□ un groupement alkyle si le substituant lui-même n'est pas un radical alkyle.

3. Composition selon l'une des revendications précédentes, **caractérisé en ce que** les radicaux R₂ à R₅ sont des radicaux cycliques ou polycycliques comprenant au moins un hétéroatome, choisi parmi l'oxygène, l'azote et le soufre, comprenant de 5 à 6 chaînons, de préférence de 5 à 6 chaînons, saturés ou non, ou aromatiques. éventuellement substitués.

4. Composition selon l'une des revendications précédentes, **caractérisé en ce que** les radicaux R₁ et R₆, identiques ou non, sont choisis parmi l'hydrogène, un radical hydroxyle, un radical alkyle en C₁-C₆, un radical monohydroxyalkyle en C₁-C₆, un radical polyhydroxyalkyle en C₂-C₈, un radical alcoxy en C₁-C₈, un radical alkylcarbonyle en C₁-C₆, un radical amino, un radical amino mono- ou di-substitué par un radical alkyle en C₁-C₆ et/ou par un radical hydroxyalkyle en C₁-C₈ ; un radical benzyle ou phényle éventuellement substitué au sens Indiqué auparavant ; un groupement nitro ; un groupement cyano.

5. Composition selon l'une des revendications précédentes, **caractérisé en ce que** les radicaux R₂ à R₆, identiques ou non, sont choisis parmi l'hydrogène ; un radical hydroxyle ; un radical thiol ; un atome d'halogène et de préférence le chlore ou le fluor ; un radical alkyle en C₁-C₆ ; un radical monohydroxyalkyle en C₁-C₆ ; un radical polyhydroxyalkyle en C₂-C₆ ; un radical alcoxy en C₁-C₆ ; un radical alkylcarbonyle en C₁-C₆ ; un radical amino ; un radical amino mono- ou di-substitué par un radical alkyle en C₁-C₆ et/ou par un radical hydroxyalkyle en C₁-C₆ ; un radical benzyle ou phényle éventuellement substitué au sens indiqué auparavant ; un groupement nitro ; un groupement cyano ; un groupement hétérocyclique.

6. Composition selon l'une des revendications précédentes **caractérisée en ce que** l'un des radicaux R₃ ou R₄ représente un radical aryle en C₆-C₁₂, substitué ou non par un radical hydroxyle ; un atome d'halogène ; un radical alcoxy en C₁-C₄ ; un radical amino ; un radical amino substitué par un ou plusieurs radicaux alkyles, identiques ou non, en C₁-C₄ ; un radical thiol.

7. Composition selon l'une quelconque des revendications précédentes **caractérisée en ce que** R₁, R₂, R₅ ou R₈ représentent un atome d'hydrogène.

8. Composition selon l'une quelconque des revendications précédentes **caractérisée en ce que** le composé de formule (I) est choisi parmi le 8,8-dicyano-3-(4'-méthoxy)-phénylheptafulvène ; le 8,8-dicyano-3-(4'-N,N-diméthylamino)phénylheptafulvène ; le 8,8-dicyano-3-phényl heptafulvène, ou leurs mélanges.

9. Composition selon l'une quelconque des revendications précédentes, **caractérisée en ce que** la teneur en composé (I) est comprise entre 0,5 et 5 % en poids par rapport au poids de la composition.

10. Composition selon l'une quelconque des revendications précédentes, **caractérisée en ce qu'**elle comprend au moins un colorent direct, fluorescent ou non.

11. Composition selon la revendication précédente, **caractérisée en ce que** le colorant direct est choisi les colorants benzéniques nitrés, les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines, diazines, dicétopyrrolopyrroles, dioxazines, diphénylamines, diphénylméthanes, dithiazines, flavanthrones, flavones, fluorindines, formazans, hydrazones, hydroxycétones, indamines, indanthrones, indigoides, Indophénols, Indoanilines, isoindolines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes.

12. Composition selon la revendication 11, **caractérisée en ce que** le colorant direct fluorescent est choisi parmi les composés fluorescents cationiques de la famille des composés azoïques, azométhiniques, méthiniques, naphtalimides, coumarines cationiques ou non, xanthénodiquinolizines, les azaxanthénes, naphtolactames, aziactones, (di)oxazines, thiazines, seuls ou en mélanges.

13. Composition selon l'une quelconque des revendications 10 à 12, **caractérisée en ce que** la teneur en colorant(s) direct(s), fluorescent(s) ou non, représente de 0,0005 à 12 % en poids par rapport au poids de la composition et encore plus préférentiellement de 0,005 à 8 % en poids par rapport au poids de la composition.

14. Composition selon l'une quelconque des revendications précédentes, **caractérisée en ce que** la composition comprend au moins une base d'oxydation éventuellement associée à un ou plusieurs coupleurs.

15. Composition selon la revendication 14, **caractérisée en ce que** la base d'oxydation est choisie parmi les o-phénylénediamines, les p-phénylènediamines, les bases doubles, les o-aminophénols, les p-aminophénols, les bases hétérocycliques, leurs sels d'addition avec un adde, ainsi que leurs mélanges.

16. Composition selon la revendication précédente, **caractérisée en ce que** la teneur en bases d'oxydation représente de 0,0005 à 12% en poids par rapport au poids de la composition, et de préférence de 0,005 à 8% en poids par rapport au poids de la composition.

17. Composition selon la revendication 14, **caractérisée en ce que** le coupleur est choisi parmi les m-aminophénols, les m-phénylènediamines, les m-diphénols, les naphtols, les coupleurs hétérocycliques, leurs sels d'addition avec un acide, ainsi que leurs mélanges.

18. Composition selon la revendication précédente, **caractérisée en ce que** la teneur en coupleurs représente de 0,0001 à 10% en poids par rapport au poids de la composition, et de préférence de 0,005 à 5% en poids par rapport au poids de la composition.

19. Composition prête à l'emploi, **caractérisée en ce qu'**elle comprend la composition selon l'une quelconque des revendications précédentes, et au moins un agent oxydant.

20. Composition prête à l'emploi selon le revendication précédente, **caractérisée en ce que** l'agent oxydant est choisi parmi le peroxyde d'hydrogène, le peroxyde d'urée, les bromates ou ferricyanures de métaux alcalins, les persels tels que les perborates et les persulfates, les enzymes, seuls ou en mélanges.

21. Procédé de teinture des fibres kératiniques, notamment humaines dans lequel on applique la composition selon l'une quelconque des revendications sur lesdites fibres sèches ou humides.

22. Procédé salon la revendication précédente, **caractérisé** an ce qu'après l'application de la composition sur les fibres, celles-ci sont séchées ou laissées à sécher.

23. Procédé selon la revendication 21, **caractérisé en ce que** l'on met en oeuvre les étapes suivantes :
a) on met en contact lesdites fibres, sèches ou humides, avec la composition selon l'une des revendications précédentes pendant une durée suffisante pour le développement de la coloration,
b) on rince éventuellement les fibres,
c) éventuellement on lave les fibres et on les rince,
d) on sèche les fibres ou on les laisse sécher.

24. Dispositif pour la mise en oeuvre du procédé selon la revendication précédente, présentant au moins un compartiment comprenant la composition selon l'une quelconque des revendications 1 à 18 et au moins un compartiment comprenant au moins un agent oxydant.

25. Utilisation de composés de formule (I) tels que définis selon l'une quelconque des revendications 1 à 12, comme colorants directs pour la coloration de fibres kératiniques, notamment humaines afin d'obtenir des nuances naturelles et chromatiques.

## Claims

1. Composition comprising, in a medium that is suitable for dyeing keratin fibres, especially human keratin fibres, from 0.1% to 20% by weight relative to the weight of the composition of at least one compound of formula (I) below: 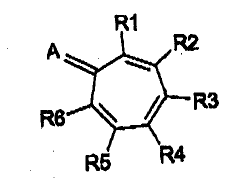 in which formula:
* R₁ and R₆, which may be identical or different, represent a hydrogen atom; an alkyl radical, an aryl radical, an alkylaryl radical or an arylalkyl radical, for which the alkyl part is C₁-C₆, linear or branched, and substituted or unsubstituted, and the aryl part is C₆-C₁₂, and substituted or unsubstituted; a halogen atom; a C₁-C₆ alkoxy radical; an amino radical; an amino radical substituted with one or more radicals, which may be identical or different, chosen from linear or branched, substituted or unsubstituted C₁-C₆ alkyl radicals; a hydroxyl radical; a radical -COOX, with X representing a hydrogen atom, an alkali metal or alkaline-earth metal or a linear or branched, substituted or unsubstituted C₁-C₆ alkyl radical; an alkylcarbonyl radical in which the alkyl part is C₁-C₆, linear or branched, and substituted or unsubstituted; a radical -SO₃X' in which X' denotes a hydrogen atom or an alkali metal or alkaline-earth metal;
* R₂, R₃, R₄ and R₅, which may be identical or different, represent a hydrogen atom or a linear or branched, substituted or unsubstituted C₁-C₆ alkyl radical; a hydroxyl radical; a C₁-C₆ alkoxy radical; a thiol radical or a C₁-C₆ alkylthio radical; an amino radical; an amino radical substituted with one or more linear or branched, substituted or unsubstituted C₁-C₆ radicals, which may be identical or different; a substituted or unsubstituted C₆-C₁₂ aryl radical, or an arylalkyl radical in which the alkyl part is C₁-C₆, linear or branched, and substituted or unsubstituted, and the aryl part is C₆-C₁₂; a substituted or unsubstituted cyclic or polycyclic radical comprising at least one heteroatom;
* A denotes a group C(R₇)(R₈) in which R₇ and R₈ denote a cyano radical.

2. Composition according to the preceding claim, **characterized in that** R₁ to R₆ are such that the substituted radicals bear one or more groups chosen from:
□ a hydroxyl group;
□ a halogen atom, preferably chlorine or fluorine;
□ a C₁-C₆ alkoxy group;
□ a monohydroxyalkoxy group in which the alkyl part is C₁-C₆, linear or branched, and substituted or unsubstituted;
□ a polyhydroxyalkoxy group, in which the alkyl part is C₂-C₆, linear or branched, and substituted or unsubstituted;
□ an amino group, which is unsubstituted or substituted with one or more linear or branched, substituted or unsubstituted C₁-C₆ alkyl radicals, which may be identical or different;
□ a thiol group;
□ an alkylthio group in which the alkyl part is C₁-C₆, linear or branched, and substituted or unsubstituted;
□ a carboxylic group in acid or salified form (for example salified with an alkali metal or a substituted or unsubstituted ammonium);
□ an alkylcarbonyl group in which the alkyl part is C₁-C₆, linear or branched, and substituted or unsubstituted;
□ an alkoxycarbonyl group in which the alkyl part is C₁-C₆, linear or branched, and substituted or unsubstituted;
□ an alkylamide group, the alkyl part of which is C₁-C₆, linear or branched, and substituted or unsubstituted;
□ an alkylcarbamyl group, the alkyl part of which is C₁-C₆, linear or branched, and substituted or unsubstituted;
□ a cyano group;
□ a nitro group;
□ a sulfonyl group;
□ an alkylsulfonyl group in which the alkyl part is C₁-C₆, linear or branched, and substituted or unsubstituted;
□ an alkylsulfonylamido group in which the alkyl part is C₁-C₆, linear or branched, and substituted or unsubstituted;
□ an alkyl group, if the substituent is itself other than an alkyl radical.

3. Composition according to either of the preceding claims, **characterized in that** the radicals R₂ to R₅ are saturated or unsaturated, or aromatic, optionally substituted, 5- to 8-membered and preferably 5- to 6-membered cyclic or polycyclic radicals comprising at least one heteroatom chosen from oxygen, nitrogen and sulfur.

4. Composition according to one of the preceding claims, **characterized in that** the radicals R₁ and R₆, which may be identical or different, are chosen from hydrogen, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a C₁-C₆ alkylcarbonyl radical, an amino radical, an amino radical mono- or disubstituted with a C₁-C₆ alkyl radical and/or with a C₁-C₆ hydroxyalkyl radical; a benzyl or phenyl radical optionally substituted in the sense indicated previously; a nitro group; a cyano group.

5. Composition according to one of the preceding claims, **characterized in that** the radicals R₂ to R₆, which may be identical or different, are chosen from hydrogen; a hydroxyl radical; a thiol radical; a halogen atom and preferably chlorine or fluorine; a C₁-C₆ alkyl radical; a C₁-C₆ monohydroxyalkyl radical; a C₂-C₆ polyhydroxyalkyl radical; a C₁-C₆ alkoxy radical; a C₁-C₆ alkylcarbonyl radical; an amino radical; an amino radical mono- or disubstituted with a C₁-C₆ alkyl radical and/or with a C₁-C₆ hydroxyalkyl radical; a benzyl or phenyl radical optionally substituted in the sense indicated previously; a nitro group; a cyano group; a heterocyclic group.

6. Composition according to one of the preceding claims, **characterized in that** one of the radicals R₃ or R₄ represents a C₆-C₁₂ aryl radical which is unsubstituted or substituted with a hydroxyl radical; a halogen atom; a C₁-C₄ alkoxy radical; an amino radical; an amino radical substituted with one or more C₁-C₄ alkyl radicals, which may be identical or different; a thiol radical.

7. Composition according to any one of the preceding claims, **characterized in that** R₁, R₂, R₅ or R₆ represent a hydrogen atom.

8. Composition according to any one of the preceding claims, **characterized in that** the compound of formula (I) is chosen from 8,8-dicyano-3-(4'-methoxy)phenylheptafulvene; 8,8-dicyano-3-(4'-N,N-dimethylamino)phenylheptafulvene; 8,8-dicyano-3-phenylheptafulvene, or mixtures thereof.

9. Composition according to any one of the preceding claims, **characterized in that** the content of compound (I) is between 0.5% and 5% by weight relative to the weight of the composition.

10. Composition according to any one of the preceding claims, **characterized in that** it comprises at least one fluorescent or non-fluorescent direct dye.

11. Composition according to the preceding claim, **characterized in that** the direct dye is chosen from nitrobenzene dyes, acridine, acridone, anthranthrone, anthrapyrimidine, anthraquinone, azine, azo, azomethine, benzanthrone, benzimidazole, benzimidazolone, benzindole, benzoxazole, benzopyran, benzothiazole, benzoquinone, bisazine, bis-isoindoline, carboxanilide, coumarin, cyanin, diazin, diketopyrrolopyrrole, dioxazine, diphenylamine, diphenylmethane, dithiazine, flavanthrone, flavone, fluorindine, formazan, hydrazone, hydroxy ketone, indamine, indanthrone, indigoid, indophenol, indoaniline, isoindoline, isoindolinone, isoviolanthrone, lactone, methine, naphthalimide, naphthanilide, naphtholactam, naphthoquinone, nitro, oxadiazole, oxazine, perilone, perinone, perylene, phenazine, phenothiazine, phthalocyanin, polyene/carotenoid, porphyrin, pyranthrone, pyrazolanthrone, pyrazolone, pyrimidinoanthrone, pyronine, quinacridone, quinoline, quinophthalone, squarane, stilbene, tetrazolium, thiazine, thioindigo, thiopyronine, triarylmethane and xanthene dyes.

12. Composition according to Claim 11, **characterized in that** the fluorescent direct dye is chosen from cationic fluorescent compounds of the family of azo, azomethine, methine, naphthalimide, cationic or non-cationic coumarin, xanthenodiquinolizine, azaxanthene, naphtholactam, azlactone, (di)oxazine and thiazine compounds, alone or as mixtures.

13. Composition according to any one of Claims 10 to 12, **characterized in that** the content of fluorescent or non-fluorescent direct dye(s) represents from 0.0005% to 12% by weight relative to the weight of the composition, and even more preferably from 0.005% to 6% by weight relative to the weight of the composition.

14. Composition according to any one of the preceding claims, **characterized in that** the composition comprises at least one oxidation base optionally combined with one or more couplers.

15. Composition according to Claim 14, **characterized in that** the oxidation base is chosen from o-phenylenediamines, p-phenylenediamines, double bases, o-aminophenols, p-aminophenols and heterocyclic bases, addition salts thereof with an acid, and also mixtures thereof.

16. Composition according to the preceding claim, **characterized in that** the content of oxidation bases represents from 0.0005% to 12% by weight relative to the weight of the composition, and preferably from 0.005% to 8% by weight relative to the weight of the composition.

17. Composition according to Claim 14, **characterized in that** the coupler is chosen from m-aminophenols, m-phenylenediamines, m-diphenols, naphthols and heterocyclic couplers, the addition salts thereof with an acid, and also mixtures thereof.

18. Composition according to the preceding claim, **characterized in that** the content of couplers represents from 0.0001% to 10% by weight relative to the weight of the composition, and preferably from 0.005% to 5% by weight relative to the weight of the composition.

19. Ready-to-use composition, **characterized in that** it comprises the composition according to any one of the preceding claims, and at least one oxidizing agent.

20. Ready-to-use composition according to the preceding claim, **characterized in that** the oxidizing agent is chosen from hydrogen peroxide, urea peroxide, alkali metal bromates or ferricyanides, persalts such as perborates and persulfates, and enzymes, alone or as mixtures.

21. Process for dyeing keratin fibres, especially human keratin fibres, in which the composition according to any one of the claims is applied to the said wet or dry fibres.

22. Process according to the preceding claim, **characterized in that**, after applying the composition to the fibres, they are dried or are left to dry.

23. Process according to Claim 21, **characterized in that** the following steps are performed:
a) the said wet or dry fibres are placed in contact with the composition according to one of the preceding claims for a time that is sufficient to develop the coloration,
b) the fibres are optionally rinsed,
c) the fibres are optionally washed and rinsed,
d) the fibres are dried or are left to dry.

24. Device for performing the process according to the preceding claim, containing at least one compartment comprising the composition according to any one of Claims 1 to 18, and at least one compartment comprising at least one oxidizing agent.

25. Use of compounds of formula (I) as defined according to any one of Claims 1 to 12, as direct dyes for dyeing keratin fibres, especially human keratin fibres, in order to obtain natural and chromatic shades.

## Patentansprüche

1. Zusammensetzung, die in einem zum Färben von Keratinfasern und insbesondere menschlichen Keratinfasern geeigneten Medium 0,1 bis 20 Gew.-% einer Verbindung der folgenden Formel (I), bezogen auf das Gesamtgewicht der Zusammensetzung, enthält: 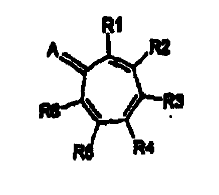 wobei in der Formel bedeuten:
• R₁ und R₆, die gleich oder verschieden sind, ein Wasserstoffatom; eine Alkylgruppe, eine Arylgruppe, eine Alkylarylgruppe, eine Arylalkylgruppe, bei denen die Alkylgruppe 1 bis 6 Kohlenstoffatome aufweist und geradkettig oder verzweigt, substituiert oder unsubstituiert vorliegt, die Arylgruppe 6 bis 12 Kohlenstoffatome aufweist und substituiert oder unsubstituiert ist; ein Halogenatom; eine C₁₋₆-Alkoxygruppe; eine Aminogruppe; eine Aminogruppe, die mit einer oder mehreren, gleichen oder voneinander verschiedenen Gruppen substituiert ist, die unter den geradkettigen oder verzweigten, substituierten oder unsubstituierten C₁-₆-Alkylgruppen ausgewählt sind; eine Hydroxygruppe; eine Gruppe -COOX, wobei X ein Wasserstoffatom, ein Alkalimetall oder ein Erdalkalimetall, eine geradkettige oder verzweigte, substituierte oder unsubstituierte C₁₋₆-Alkylgruppe bedeutet; eine Alkylcarbonylgruppe, bei der die Alkylgruppe 1 bis 6 Kohlenstoffatome aufweist und geradkettig oder verzweigt, substituiert oder unsubstituiert vorliegt; eine Gruppe -SO₃X', wobei X' ein Wasserstoffatom, ein Alkalimetall oder ein Erdalkalimetall bedeutet;
• R₂, R₃, R₄ und R₅, die gleich oder verschieden sind, ein Wasserstoffatom, eine geradkettige oder verzweigte, substituierte oder unsubstituierte C₁₋₆-Alkylgruppe; eine Hydroxygruppe; eine C₁₋₆-Alkoxygruppe; eine Thiogruppe, eine C₁₋₆-Alkylthiogruppe; eine Aminogruppe; eine Aminogruppe, die mit einer oder mehreren, geradkettigen oder verzweigten, substituierten oder unsubstituierten, gleichen oder voneinander verschiedenen C₁₋₆-Alkylgruppen substituiert ist; eine substituierte oder unsubstituierte C₆₋₁₂-Arylgrupe, eine Alkylarylgruppe, wobei die Alkylgruppe 1 bis 6 Kohlenstoffatome aufweist und geradkettig oder verzweigt, substituiert oder unsubstituiert ist und die Arylgruppe 6 bis 12 Kohlenstoffatome aufweist; eine cyclische oder polycyclische Gruppe, die gegebenenfalls substituiert ist und mindestens ein Heteroatom enthält;
• A eine Gruppe C(R₇)(R₈), wobei R₇ und R₈ eine Cyanogruppe bedeuten.

2. Zusammensetzung nach dem vorhergehenden Anspruch, **dadurch gekennzeichnet, dass** R₁ bis R₆ so ausgewählt sind, dass die substituierten Gruppen eine oder mehrere Gruppen tragen, die ausgewählt sind unter:
□ einer Hydroxygruppe;
□ einem Halogenatom und vorzugsweise Chlor, Fluor;
□ einer C₁₋₆-Alkoxygruppe;
□ einer Monohydroxyalkoxygruppe, bei der die Alkylgruppe 1 bis 6 Kohlenstoffatome aufweist und geradkettig oder verzweigt, substituiert oder unsubstituiert ist;
□ einer Polyhydroxyalkoxygruppe, bei die Alkylgruppe 2 bis 6 Kohlenstoffatome aufweist und geradkettig oder verzweigt, substituiert oder unsubstituiert ist;
□ einer Aminogruppe, die gegebenenfalls mit einer oder mehreren geradkettigen oder verzweigten, substituierten oder unsubstituierten, gleichen oder voneinander verschiedenen C₁₋₆-Alkylgruppen substituiert ist;
□ einer Thiogruppe;
□ einer Alkylthiogruppe, bei der die Alkylgruppe 1 bis 6 Kohlenstoffatome aufweist und geradkettig oder verzweigt, substituiert oder unsubstituiert ist;
□ einer Carboxygruppe in Form der Säure oder als Salz (beispielsweise mit einem Alkalimetall oder einer substituierten oder unsubstituierten Ammoniumgruppe);
□ einer Alkylcarbonylgruppe, bei der die Alkylgruppe 1 bis 6 Kohlenstoffatome aufweist und geradkettig oder verzweigt, substituiert oder unsubstituiert ist;
□ einer Alkoxycarbonylgrupe, bei der die Alkylgruppe 1 bis 6 Kohlenstoffatome aufweist und geradkettig oder verzweigt, substituiert oder unsubstituiert ist;
□ einer Alkylamidgruppe, bei der die Alkylgruppe 1 bis 6 Kohlenstoffatome aufweist und geradkettig oder verzweigt, substituiert oder unsubstituiert ist;
□ einer Alkylcarbamoylgruppe, bei der die Alkylgruppe 1 bis 6 Kohlenstoffatome aufweist und geradkettig oder verzweigt, substituiert oder unsubstituiert ist;
□ einer Cyanogruppe;
□ einer Nitrogruppe;
□ einer Sulfonylgruppe;
□ einer Alkylsulfonylgruppe, bei der die Alkylgruppe 1 bis 6 Kohlenstoffatome aufweist und geradkettig oder verzweigt, substituiert oder unsubstituiert ist;
□ einer Alkylsulfonylamidogruppe, bei der die Alkylgruppe 1 bis 6 Kohlenstoffatome aufweist und geradkettig oder verzweigt, substituiert oder unsubstituiert ist;
□ einer Alkylgruppe, solange der Substituent selbst keine Alkylgruppe ist.

3. Zusammensetzung nach einem der vorhergehenden Ansprüche, **dadurch gekennzeichnet, dass** die Gruppen R₂ bis R₅ cyclische oder polycyclische Gruppe sind, die mindestens ein Heteroatom enthalten, das unter Sauerstoff, Stickstoff und Schwefel ausgewählt ist, wobei sie 5- bis 6-gliedrig sind und vorzugsweise 5- bis 6-gliedrige, gesättigte oder ungesättigte oder aromatische, gegebenenfalls substituierte Gruppierungen sind.

4. Zusammensetzung nach einem der vorhergehenden Ansprüche, **dadurch gekennzeichnet, dass** die Gruppen R₁ und R₆, die gleich oder verschieden sind, unter Wasserstoff, Hydroxy, C₁-₆-Alkyl, C₁₋₆-Monohydroxyalkyl, C₂₋₆-Polyhydroxyalkyl, C₁₋₆-Alkoxy, C₁₋₆-Alkylcarbonyl, Amino, einer mit C₁₋₆-Alkyl und/oder C₁₋₆-Hydroxyalkyl mono- oder disubstituierten Aminogruppe; Benzyl oder Phenyl, die gegebenenfalls im oben angegebenen Sinn substituiert sind; Nitro; Cyano ausgewählt sind.

5. Zusammensetzung nach einem der vorhergehenden Ansprüche, **dadurch gekennzeichnet, dass** die Gruppen R₂ bis R₅, die gleich oder verschieden sind, unter Wasserstoff; Hydroxy, Thiol; einem Halogenatom und vorzugsweise Chlor oder Fluor; C₁₋₆-Alkyl; C₁₋₆-Monohydroxyalkyl; C₂₋₆-Polyhydroxyalkyl; C₁₋₆-Alkoxy; C₁₋₆-Alkylcarbonyl; Amino, einer mit C₁₋₆-Alkyl und/oder C₁₋₆-Hydroxyalkyl mono- oder disubstituierten Aminogruppe; Benzyl oder Phenyl, die wie gegebenenfalls wie oben angegeben substituiert sind; Nitro, Cyano; einer heterocyclischen Gruppe ausgewählt sind.

6. Zusammensetzung nach einem der vorhergehenden Ansprüche, **dadurch gekennzeichnet, dass** die Gruppen R₃ oder R₄ eine C₆-₁₂-Arylgruppe bedeuten, die gegebenenfalls mit einer Hydroxygruppe substituiert ist; ein Halogenatom; C₁₋₄-Alkoxy; Amino; eine Aminogruppe, die mit einer oder mehreren, gleichen oder voneinander verschiedenen C₁₋₄-Alkylgruppen substituiert ist; eine Thiogruppe bedeuten.

7. Zusammensetzung nach einem der vorhergehenden Ansprüche, **dadurch gekennzeichnet, dass** R₁, R₂, R₅ oder R₆ ein Hydrogenatom bedeuten.

8. Zusammensetzung nach einem der vorhergehenden Ansprüche, **dadurch gekennzeichnet, dass** die Verbindung der Formel (I) unter 8,8-Dicyano-3-(4'-methoxy)-phenylheptafulven; 8,8-Dicyano-3-(4'-N,N-dimethylamino)-phenylheptafulven; 8,8-Dicyano-3-phenyl-heptafulven oder deren Gemischen ausgewählt ist.

9. Zusammensetzung nach einem der vorhergehenden Ansprüche, **dadurch gekennzeichnet, dass** der Mengenanteil der Verbindung (I) im Bereich von 0,5 bis 5 Gew.-%, bezogen auf das Gesamtgewicht der Zusammensetzung, liegt.

10. Zusammensetzung nach einem der vorhergehenden Ansprüche, **dadurch gekennzeichnet, dass** sie mindestens einen fluoreszierenden oder nichtfluoreszierenden Direktfarbstoff enthält.

11. Zusammensetzung nach dem vorhergehenden Anspruch, **dadurch gekennzeichnet, dass** der Direktfarbstoff ausgewählt ist unter nitrierten Benzol-Farbstoffen, Acridin-Farbstoffen, Acridon-Farbstoffen, Anthranthron-Farbstoffen, Anthrapyrimidin-Farbstoffen, Anthrachinon-Farbstoffen, Azin-Farbstoffen, Azofarbstoffen Azomethin-Farbstoffen Benzanthron-Farbstoffen Benzimidazol-Farbstoffen, Benzimidazolon-Farbstoffen, Benzindol-Farbstoffen, Benzoxazol-Farbstoffen, Benzopyran-Farbstoffen, Benothiazol-Farbstoffen, Benzochinon-Farbstoffen, Bisazin-Farbstoffen, Bisisoindolin-Farbstoffen, Carboxanilid-Farbstoffen, Cumarin-Farbstoffen, Cyanin-Farbstoffen, Diazin-Farbstoffen, Diketopyrrolopyrrol-Farbstoffen, Dioxazin-Farbstoffen, Diphenylamin-Farbstoffen, Diphenylmethan-Farbstoffen, Dithiazin-Farbstoffen, Flavanthron-Farbstoffen, Flavon-Farbstoffen, Fluoridin-Farbstoffen, Formazan-Farbstoffen, Hydrazon-Farbstoffen, Hydroxyketon-Farbstoffen, Indamin-Farbstoffen, Indanthron-Farbstoffen, Indigoiden, Indophenol-Farbstoffen, Indoanilin-Farbstoffen, Isoindolin-Farbstoffen, Isoindolin-Farbstoffen, Isoindolinon-Farbstoffen, Isoviolanthron-Farbstoffen, Lacton-Farbstoffen, Methin-Farbstoffen, Naphthalimid-Farbstoffen, Naphthanilid-Farbstoffen, Naphtholactam-Farbstoffen, Naphthochinon-Farbstoffen, Nitro-Farbstoffen, Oxadiazol-Farbstoffen, Oxazin-Farbstoffen, Perilon-Farbstoffen, Perinon-Farbstoffen, Perylen-Farbstoffen, Phenanzin-Farbstoffen, Phenothiazin-Farbstoffen, Phthalocyanin-Farbstoffen, Polyenen/Carotinoiden, Porphyrin-Farbstoffen, Pyranthron-Farbstoffen, Pyrazolanthron-Farbstoffen, Pyrazolon-Farbstoffen, Pyrimidinoanthron-Farbstoffen, Pyronin-Farbstoffen, Chinacridon-Farbstoffen, Chinolin-Farbstoffen, Chinophthalon-Farbstoffen, Squaran-Farbstoffen, Stilben-Farbstoffen, Tetrazolium-Farbstoffen, Thiazin-Farbstoffen, Thioindigo-Farbstoffen, Thiopyronin-Farbstoffen, Triarylmethan-Farbstoffen, Xanthen-Farbstoffen.

12. Zusammensetzung nach Anspruch 11, **dadurch gekennzeichnet, dass** der fluoreszierende Direktfarbstoff, einzeln oder in Form von Gemischen, unter den kationischen fluoreszierenden Verbindungen aus der Gruppe der Azoverbindungen, Azomethine, Methine, Naphthalimide, kationischen oder nicht kationischen Cumarine, Xanthenodichinolizine, Azaxanthene, Naphtholactame, Azlactone, (Di)oxazine, Thiazine ausgewählt ist.

13. Zusammensetzung nach einem der Anspräche 10 bis 12, **dadurch gekennzeichnet, dass** der Mengenanteil des fluoreszierenden oder nicht fluoreszierenden direktziehenden Farbstoffes oder der fluoreszierenden oder nicht fluoreszierenden direktziehenden Farbstoffe 0,0005 bis 12 Gew.-%, bezogen auf das Gesamtgewicht der Zusammensetzung, und noch bevorzugter 0,005 bis 6 Gew.-%, bezogen auf das Gewicht der Zusammensetzung, ausmacht.

14. Zusammensetzung nach einem der vorhergehenden Ansprüche, **dadurch gekennzeichnet, dass** die Zusammensetzung mindestens eine Oxidationsbase gegebenenfalls in Kombination mit einem oder mehreren Kupplern enthält.

15. Zusammensetzung nach Anspruch 14, **dadurch gekennzeichnet, dass** die Oxidationsbase unter den o-Phenylendiaminen, p-Phenylendiaminen, Doppelbasen, o-Aminophenolen, p-Aminophenolen, heterocyclischen Basen, ihren Additionssalzen mit einer Säure sowie ihren Gemischen ausgewählt ist.

16. Zusammensetzung nach dem vorhergehenden Anspruch, **dadurch gekennzeichnet, dass** der Mengenanteil der Oxiationsbasen 0,0005 bis 12 Gew.-%, bezogen auf das Gesamtgewicht der Zusammensetzung, und vorzugsweise 0,005 bis 8 Gew.-%, bezogen auf das Gesamtgewicht der Zusammensetzung, ausmacht.

17. Zusammensetzung nach Anspruch 14, **dadurch gekennzeichnet, dass** der Kuppler unter den *m*-Aminophenolen, *m*-Phenylendiaminen, *m*-Dihydroxybenzolen, Naphtholen, heterocyclischen Kupplern, deren Additionssalzen mit einer Säure sowie ihren Gemischen ausgewählt ist.

18. Zusammensetzung nach dem vorhergehenden Anspruch, **dadurch gekennzeichnet, dass** der Mengenanteil der Kuppler 0,0001 bis 10 Gew.-%, bezogen auf das Gesamtgewicht der Zusammensetzung, und vorzugsweise 0,005 bis 5 Gew.-%, bezogen auf das Gesamtgewicht der Zusammensetzung, ausmacht.

19. Gebrauchsfertige Zusammensetzung, **dadurch gekennzeichnet, dass** sie die Zusammensetzung nach einem der vorhergehenden Ansprüche und mindestens ein Oxidationsmittel enthält.

20. Gebrauchsfertige Zusammensetzung nach dem vorhergehenden Anspruch, **dadurch gekennzeichnet, dass** das Oxidationsmittel einzeln oder in Form von Gemischen unter Wasserstoffperoxid, Harnstoffperoxid, Alkalimetallbromaten, Alkalimetallferricyaniden, Salzen von Persäuren, wie Perboraten und Persulfaten, Enzymen ausgewählt ist.

21. Verfahren zum Färben von Keratinfasern und insbesondere menschlichen Keratinfasern, bei dem die Zusammensetzung nach einem der vorhergehenden Ansprüche auf die trockenen oder feuchten Fasern aufgetragen wird.

22. Verfahren nach dem vorhergehenden Anspruch, **dadurch gekennzeichnet, dass** die Fasern nach dem Aufbringen der Zusammensetzung auf die Fasern getrocknet oder trocknen gelassen werden.

23. Verfahren nach Anspruch 21, **dadurch gekennzeichnet, dass** die folgenden Schritte durchgeführt werden:
a) die trockenen oder feuchten Fasern werden mit der Zusammensetzung nach einem der vorhergehenden Ansprüche während einer Zeitspanne, die für die Bildung der Färbung ausreichend ist, in Kontakt gebracht,
b) die Fasern werden gegebenenfalls gespült,
c) die Fasern werden gegebenenfalls gewaschen und gespült,
d) die Fasern werden getrocknet oder trocknen gelassen.

24. Vorrichtung für die Durchführung des Verfahrens nach dem vorhergehenden Anspruch, die mindestens eine Abteilung mit einer Zusammensetzung nach einem der Ansprüche 1 bis 18 und mindestens eine Abteilung mit mindestens einem Oxidationsmittel aufweist.

25. Verwendung der Verbindungen der Formel (I) nach einem der Ansprüche 1 bis 12 als Direktfarbstoffe zum Färben von Keratinfasern und insbesondere menschlichen Keratinfasern, um natürliche und chromatische Farbnuancen zu erzeugen.
